(19) Europäisches Patentamt
European Patent Office
Office européen des brevets

(11) **EP 4 730 338 A1**

(12) **EUROPEAN PATENT APPLICATION**
published in accordance with Art. 153(4) EPC

(43) Date of publication:
**22.04.2026 Bulletin 2026/17**

(21) Application number: **23941873.4**

(22) Date of filing: **19.06.2023**

(51) International Patent Classification (IPC):
***G16B 20/30*** (2019.01)

(52) Cooperative Patent Classification (CPC):
**G16B 20/30**

(86) International application number:
**PCT/CN2023/101131**

(87) International publication number:
**WO 2024/259566 (26.12.2024 Gazette 2024/52)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Tianjin Medical Laboratory, BGI**
Tianjin 300308 (CN)
• **BGI Tianjin**
Tianjin 300308 (CN)

(72) Inventors:
• **TANG, Fei**
Tianjin 300308 (CN)
• **WANG, Zhonghua**
Tianjin 300308 (CN)
• **SUN, Yan**
Tianjin 300308 (CN)
• **SONG, Lijie**
Tianjin 300308 (CN)

(74) Representative: **Dehns**
**10 Old Bailey**
**London EC4M 7NG (GB)**

(54) **TRAINING METHOD AND APPARATUS FOR LOSS OF HETEROZYGOSITY REGION DETECTION MODEL, DEVICE, AND MEDIUM**

(57)    Embodiments of the present disclosure provide a method for training a loss of heterozygosity region detection model, an apparatus, a device and a medium, relating to the technical field of computers. The method includes: acquiring first data; determining information values of each region of N regions in a chromosome corresponding to the first data, wherein the information values include mean value information and variance information of the above regions; the N regions including heterozygous regions and the LOH regions, where N is a positive integer; determining feature information of the N regions, and determining an input feature matrix based on the feature information of the N regions and the information values of the N regions, wherein the feature information includes the information of the LOH regions corresponding to the N regions and an association relationship between the information values of each region; and performing iterative training on an initial model based on the input feature matrix to obtain a LOH region detection model, wherein the initial model is determined based on a self-attention allocation algorithm.

Fig. 1

## Description

### FIELD

The present disclosure relates generally to the technical field of computers, in particular to a method for training a loss of heterozygosity region detection model, an apparatus, a device and a medium.

### BACKGROUND

Loss of Heterozygosity (LOH) is a common form of genomic variation, characterized by the loss or mutation of an allele, resulting in an originally heterozygous locus becoming homozygous. This generally affects the entire gene and the adjacent chromosomal regions, and a large number of genetic diseases are caused by LOH regions in chromosomes.

Because the LOH regions are essentially regions composed of multiple continuous Single Nucleotide Polymorphisms (SNP) loci and the number of the SNP loci contained in different LOH regions is also different, to accurately detect the LOH regions, high-depth genome sequencing data needs to be detected. For example, the LOH region is detected by the Chromosome Microarray (CMA) technology, which obtains a large amount of data information. Subsequently, different thresholds need to be set based on the characteristics of different LOH regions to screen out possible LOH regions. At the same time, a large number of literatures or databases need to be consulted to annotate the data for the screened information, and finally, a reasonable result report is obtained.

However, CMA, as a high-throughput and high-resolution screening technology, obtains a large amount of LOH information on the premise of ensuring data accuracy. It can be seen from the above process that it requires lengthy analysis steps, which consumes a lot of time cost and labor costs, thereby leading to low detection efficiency and can be popularized.

### SUMMARY

In view of the above defects or deficiencies in the prior art, it is expected to provide a method for training a loss of heterozygosity region detection model, an apparatus, a device and a medium, which can solve the problems of lengthy steps, consumption of a lot of time cost and labor cost of the current methods for detecting the LOH regions, and can greatly improve the detection efficiency.

In a first aspect, a method for training a loss of heterozygosity region detection model is provided, the method includes:

acquiring first data, wherein the first data is low-depth genome sequencing data acquired based on high-depth genome sequencing data; the first data including M types of low-depth genome sequencing data, where M is a positive integer; and the high-depth genome sequencing data including data of LOH regions;

determining information values of each region of N regions in a chromosome corresponding to the first data, wherein the information values include mean value information and variance information of the region; the N regions including heterozygous regions and the LOH regions, where N is a positive integer;

determining feature information of the N regions, and determining an input feature matrix based on the feature information of the N regions and the information values of the N regions, wherein the feature information includes information of the LOH regions corresponding to the N regions and an association relationship between the information values of each region of the N regions; and

performing iterative training on an initial model based on the input feature matrix to obtain a LOH region detection model, wherein the initial model is determined based on a self-attention allocation algorithm.

In the present disclosure, one or several sets of different low-depth genome sequencing data obtained based on the high-depth genome sequencing data are obtained at first (the high-depth genome sequencing data includes the data of the LOH regions); then, information values (mean value information and variance information) of the LOH regions in all regions (that is, N regions) in the chromosomes corresponding to the first data are determined; next, information of the LOH regions corresponding to the N regions and an association relationship between the information values of the N regions are determined, and an input feature matrix for training is determined based on the information of the LOH regions and the association relationship between the information values of each region; and finally, the initial model is iteratively trained based on the input feature matrix to obtain a LOH region detection model. In this way, by acquiring the low-depth genome sequencing data from the high-depth genome sequencing data with known LOH regions, and determining the information value and the feature information of the above low-depth genome sequencing data, then obtaining a training sample, i.e., the input feature matrix, and performing training on the model by using the input feature matrix, the LOH region detection model is finally obtained. Therefore, the LOH region detection model can accurately detect the LOH regions in the chromosomes corresponding to the genome sequencing data by using the low-depth genome sequencing data in the subsequent use process, and can accurately detect the LOH regions without the high-depth genome sequencing data for detection or other complex high-throughput detection modes, thereby greatly improving the detection efficiency.

In a second aspect, an apparatus for training a loss of heterozygosity region detection model is provided, which includes:

an acquisition module configured to acquire first data in a first format, wherein the first data is low-depth genome sequencing data acquired based on high-depth genome sequencing data; the first data including M types of low-depth genome sequencing data, where M is a positive integer; and the high-depth genome sequencing data including the data of LOH regions;

a determination module configured to determine information values of each region of N regions in a chromosome corresponding to the first data acquired by the acquisition module, wherein the information values include mean value information and variance information of the above regions; the N regions including heterozygous regions and the LOH regions, where N is a positive integer;

a determination module configured to: determine feature information of the N regions, and determine an input feature matrix based on the feature information of the N regions and the information values of the N regions, wherein the feature information includes information of the LOH regions corresponding to the N regions and an association relationship between the information values of each region of the N regions; and

a training module configured to perform iterative training on an initial model based on the input feature matrix determined by the determination module to obtain a LOH region detection model, wherein the initial model is determined based on a self-attention allocation algorithm.

In a third aspect, a computer device is provided, including a memory, a processor and a computer program stored on the memory and executable on the processor, wherein the computer program, when executed by a processor, implements the method described in the first aspect.

In a fourth aspect, a computer-readable storage medium is provided, having a computer program stored thereon, wherein the computer program, when executed by a processor, implements the method described in the first aspect.

In a fifth aspect, a computer program product is provided, including instructions, wherein the instructions, when executed by a processor, implement the method described in the first aspect.

Appended aspects and advantages of the present disclosure will be partially provided in the following description. Parts will become apparent from the following description, or will be known through the practice of the present disclosure.

**BRIEF DESCRIPTION OF DRAWINGS**

By reading the detailed description of the non-limiting embodiments with reference to the following drawings, other features, purposes and advantages of the present disclosure will become more apparent.

Fig. 1 is a flow chart of a training method for a loss of heterozygosity region detection model provided by an embodiment of the present disclosure;

Fig. 2 is a schematic diagram of a training process of the method for training the loss of heterozygosity region detection model provided by an embodiment of the present disclosure;

Fig. 3 is a schematic diagram of the structure of an apparatus for training a loss of heterozygosity region detection model provided by an embodiment of the present disclosure;

Fig. 4 is a schematic diagram of the structure of a computer device provided by an embodiment of the present disclosure.

**DESCRIPTION OF EMBODIMENTS**

The present disclosure will be further described in detail below in combination with the drawings and the embodiments. It should be understood that the specific embodiments described herein are only used for explaining the relevant present disclosure, not used for limiting the present disclosure. In addition, it should be noted that for ease of description, only portions related to the present disclosure are shown in the drawings.

It should be explained that the embodiments in the present disclosure and the features in the embodiments can be mutually combined without conflict. The present disclosure will be described in detail below by reference to the drawings and in conjunction with the embodiments.

The professional terms appearing in the present disclosure are explained as follows:

1. LOH

LOH is a chromosomal event that can cause the loss of an entire gene and the adjacent chromosomal region. If SNPs are present in the paternal and maternal genomes, then two alleles of the offspring (one comes from the male parent and one comes from the female parent) have the regions of these SNPs, and these regions are heterozygous. However, if the gene copy of one of the parents including such a region is lost, there will be only one copy in this region, so this region will lose heterozygosity, i.e., LOH. In short, if the copy of a gene from the male parent or the female parent is lost, then the region with the SNP cannot show a heterozygous state.

LOH is loss of heterozygosity, which refers to a phenomenon of the loss or mutation of one of the two alleles in a chromosome on a particular region, resulting in the retention of only one allele in the region.

2. SNP

SNP (full name: Single Nucleotide Polymorphisms) mainly refers to the DNA sequence polymorphism caused by the mutation of a single nucleotide at a genome level, including the transition and transversion of a single base. SNP localization classification can be divided into three categories: a gene coding region, a gene periphery and an intergenic SNP. SNP location regions include a UTR region, an exon or intron region of the coding region, a variable splice site, etc.

The application scenarios of the present disclosure are described as follows:

Loss of Heterozygosity (LOH) is a kind of genomic variation, which is manifested as that one allele of a pair of homologous chromosomes is deleted or mutated, while the other allele remains intact. In 1929, the genetic mechanism of the LOH was first explained by studying the X-ray-induced mutation site of *Drosophila melanogaster.* LOH is ubiquitous in cancer and can lead to the inactivation of cancer suppressor genes, thereby promoting the development of cancer. Potential mechanisms of LOH include chromosome loss, partial chromosome loss, and gene conversion. In recent years, LOH has become an important research hotspot in the field of gene detection. It is now recognized that LOH is associated with many genetic diseases, including Mendelian recessive genetic diseases. The presence of LOH on chromosomes can indicate the possibility of Uniparental Disomy (UPD), which occurs when both chromosomes of a homologous pair are inherited from the same parent. When UPD appears on specific chromosomes, it will cause related diseases due to genetic imprinting effects. A large number of studies have shown that the risk of Mendelian recessive genetic diseases in the LOH regions is significantly increased.

In recent years, with the continuous development of the high-throughput sequencing technology, the Whole Genome Sequencing (WGS) technology has been widely used in the field of disease prevention and treatment, such as genetic diseases, rare syndromes and complex diseases. In the process of clinical detection, due to the low cost of apoblema genetic detection (CNV-seq) and the detection accuracy of copy number abnormalities, CNV-seq is widely used in low-depth WGS detection and plays a very important role in the field of prenatal screening. However, although CNV-seq can detect copy number abnormalities and chromosome number abnormalities, the detection of the LOH regions is not satisfactory. This defect is caused by the generally low data depth of CNV-seq. In the process of using this detection method, corresponding probes will be set for the SNP region of high frequency alleles in the genome region, and the LOH region cannot be accurately detected by the method of LOH detection by detecting SNP homozygous or heterozygosis, and there are a lot of missed detections.

In addition, the method for detecting LOH regions in the prior art further includes: Short Tandem Repeat (STR) detection method, methylation detection (MS-PCR/MS-MLPA) method, Chromosome Microarray (CMA) detection method, and bioinformatics software (PLINK) detection method.

Wherein the bioinformatics software refers to: PLINK uses an observation method, and PLINK uses a fixed-size sliding window to scan each chromosome to find consecutive homozygous SNP. PLINK first calculates the proportion of sliding windows that contain a complete homozygous SNP, and if the proportion exceeds a preset threshold, the SNP is considered to be in a fragment of LOH. A certain number of lost or heterozygous SNPs can be specified in each sliding window to include genotypic errors, failures, or rare variants. Finally, if the number of consecutive homozygous SNPs in a fragment exceeds a number or distance threshold (the number of SNPs or the distance between chromosomes), then the fragment is identified as a LOH.

Among the above methods, STR detection needs to select highly polymorphic STR markers based on the detection purpose and the genome location, which limits the detection method and makes the detection cost high. The MS-PCR/MS-MLPA detection method consumes a long time, so the time cost is high.

At present, the most ideal technology for detecting LOH is the Chromosome Microarray (CMA) detection method. However, as a high-throughput and high-resolution screening technology, the CMA obtains a large amount of LOH information on the premise of ensuring data accuracy. Different thresholds need to be set based on different purposes for screening. Meanwhile, a large number of literatures or databases need to be consulted to annotate the data for the screened information, to finally obtain a reasonable result report.

The bioinformatics software PLINK is not accurate enough in clinical data, and there are cases of missed detection and false positive. The reason is that when the sequencing depth is too low, if only SNP loci are counted, even the truth or falsity of the SNP obtained cannot be determined, let alone the LOH region.

Therefore, a simple, fast and efficient method for detecting LOH regions is needed to solve the problem that the current method for detecting LOH regions either has lengthy steps, and consumes a lot of time cost and labor cost, or fails to detect the LOH regions accurately.

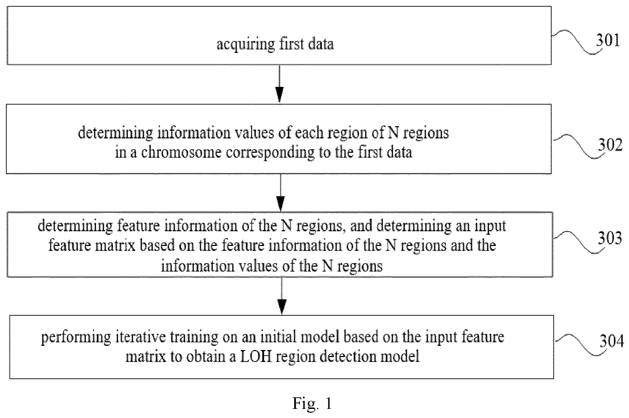

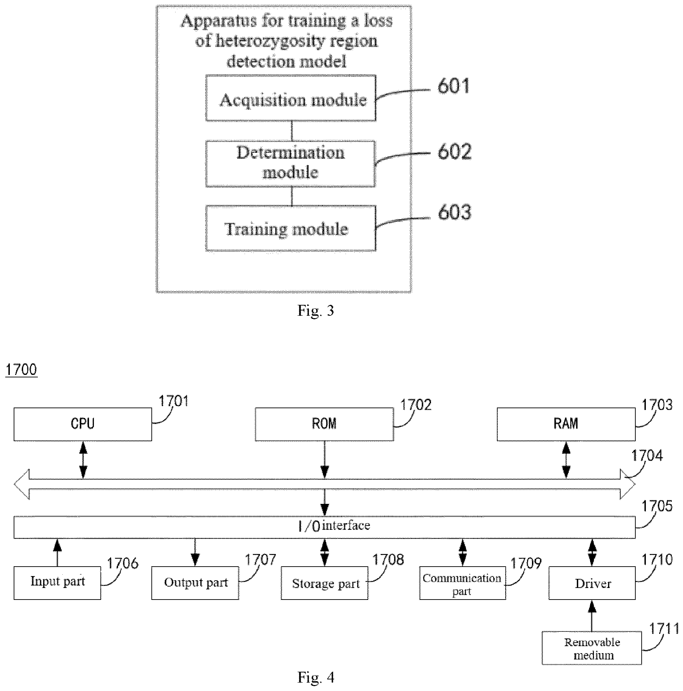

Fig. 1 is a flow chart of a method for training a loss of heterozygosity region detection model provided by an embodiment of the present disclosure. The executing subject of the method may be a computer or other terminals. As shown in Fig. 3, the method includes the following steps:

301. Acquiring the first data.

In an embodiment of the present disclosure, the first data is low-depth genome sequencing data obtained based on high-depth genome sequencing data.

In an embodiment of the present disclosure, the first data includes M types of low-depth genome sequencing data, where M is a positive integer.

In the embodiment of the present disclosure, the high-depth genome sequencing data includes data of LOH regions.

It can be understood that the depth above refers to the sequencing depth. Generally, in the process of measuring the genome sequencing data on chromosomes, due to the large amount of gene data on each chromosome, it needs to be measured at a certain depth. Different measurement depths can be used for different measurement purposes or different measurement conditions.

Exemplarily, the above M types of low-depth genome sequencing data can be one or multiple.

In one possible embodiment, when the above M types of low-depth genome sequencing data are multiple, the M types of low-depth genome sequencing data may include any or all of 3X, 1X, 0.5X and 0.1X genome sequencing data.

In one possible embodiment, when the above M types of low-depth genome sequencing data are multiple, the M types of low-depth genome sequencing data may include any of 3X, 1X, 0.5X and 0.1X genome sequencing data.

In the embodiment of the present disclosure, the first data can be extracted from the high-depth genome sequencing data. For example, the first data includes 3X, 1X, 0.5X and 0.1X genome sequencing data, which are extracted from 30X high-depth genome sequencing data, respectively.

In an embodiment of the present disclosure, the first data is genome sequencing data in a first format.

In an embodiment of the present disclosure, the first format refers to the data format of the first data, e.g., BAM format, fastq format, cram format and sam format.

It can be understood that the above high-depth genome sequencing data includes the data of the LOH regions, because it is used as the source data of the subsequent training samples. Since the purpose of the present disclosure is to train a detection model that can accurately identify the LOH regions, it is necessary to use the genome sequencing data with the LOH regions and know the information of the LOH regions (locations and sizes of the LOH regions, etc.) in the genome sequencing data.

302. Determining information values of each region of N regions in a chromosome corresponding to the first data.

In an embodiment of the present disclosure, the information value includes mean value information and variance information of the above region; the N regions include heterozygous regions and the LOH regions; where N is a positive integer.

In an embodiment of the present disclosure, the above N regions refer to: the chromosome is observed within a preset unit range, so that the chromosome corresponding to the first data is divided into several regions, wherein N regions include both heterozygous regions and LOH regions. Further, the LOH regions are homozygous regions. It should be noted that, the above N regions including the heterozygous regions and the LOH regions is known before the training is started.

For example, if the preset unit range is 10 KB, a 10 KB is taken as the observation unit on the chromosome, and the loci on the chromosome corresponding to the first data within the 10 KB unit range are detected and observed successively. The locus conditions of the SNP loci for the N regions of the chromosome contained in the N regions are finally determined. The above locus conditions refer to whether the locus belongs to the LOH region or the non-LOH region.

In an embodiment of the present disclosure, after the N regions are determined, the SNP loci in each region of the N regions are calculated, and the mean value information and the variance information of the SNP loci in each region of the N regions are finally obtained.

In one example, the above mean value information can be the mean value of the log-likelihood ratio, and the above variance can be the variance of the log-likelihood ratio.

303. Determining feature information of the N regions, and determining an input feature matrix based on the feature information of the N regions and the information values of the N regions.

In an embodiment of the present disclosure, the feature information above includes the information of the LOH regions corresponding to the N regions and an association relationship between the information values of each region of the N regions.

In an embodiment of the present disclosure, the information of the LOH regions corresponding to the N regions refers to whether the information of the identified LOH region is included at the central locus of each region of the N regions.

In one example, the information of the LOH regions corresponding to the N regions can be in coded form; for example, 0 represents a region without LOH and 1 represents a region with LOH.

In an embodiment of the present disclosure, the association relationship between the information values of each region refers to: each region of the N regions includes a large amount of mean value information and variance information, the relationship between the mean value information and the variance information in each region and between the regions needs to be observed and recorded, and finally the association relationship between the information values of each region of the N regions is formed.

It can be understood that from the above content, the LOH region is essentially composed of multiple homozygous SNP loci. On the contrary, the region where the multiple homozygous SNP loci are located is not necessarily the LOH region, which may be a normal genetic phenomenon. The LOH region has specificity. Therefore, in the prior arts of the present disclosure, the detection accuracy of the LOH region based on the low-depth genome sequencing data is very low. Therefore, after determining the N regions where the SNP loci are located, the relationship between different SNP loci should be further analyzed, that is, the information of the LOH regions corresponding to the N regions and the association relationship between the information values of each region of the N regions should be analyzed, so as to enrich the information of the training samples to ensure that the model is fully trained when these samples are used to generate the final training samples for model training. Thus, accurate detection can be achieved when the model is used to identify and detect the LOH region in the future.

In an embodiment of the present disclosure, the mode for determining the feature information above may be the mode of extracting by using a sliding window. That is, the above feature information can be extracted by sliding on the chromosome corresponding to the first data by using a sliding window.

Exemplarily, the above sliding window can be preset in advance or can be customized. For example, the sliding window can be a sliding window with a size of 2.5 MB, and the sliding step length can be consistent with the region size of each region of the N regions. If the region size of each region is 10 kb, the sliding step length is 10 kb.

In an embodiment of the present disclosure, the input feature matrix is a training sample for training the LOH region detection model.

304. Performing iterative training on an initial model based on the input feature matrix to obtain a LOH region detection model.

In an embodiment of the present disclosure, the initial model is determined based on a self-attention allocation algorithm.

In an embodiment, the initial model may be a Bidirectional Gated Recurrent Unit (BGRU)-based model.

It can be understood that the self-attention allocation algorithm includes a self-attention mechanism and other algorithms expanded based on self-attention. In general, the algorithm is composed of an input layer, a self-attention layer, a standardization layer, a hidden layer and an output layer of one neuron. In the process of processing the input of the input feature matrix by the self-attention distribution algorithm, the input feature matrix firstly passes through the input layer, then through the self-attention layer, and then through the standardization layer, then through the hidden layer of 64 neurons to the output layer of one neuron; and after output, a training is completed.

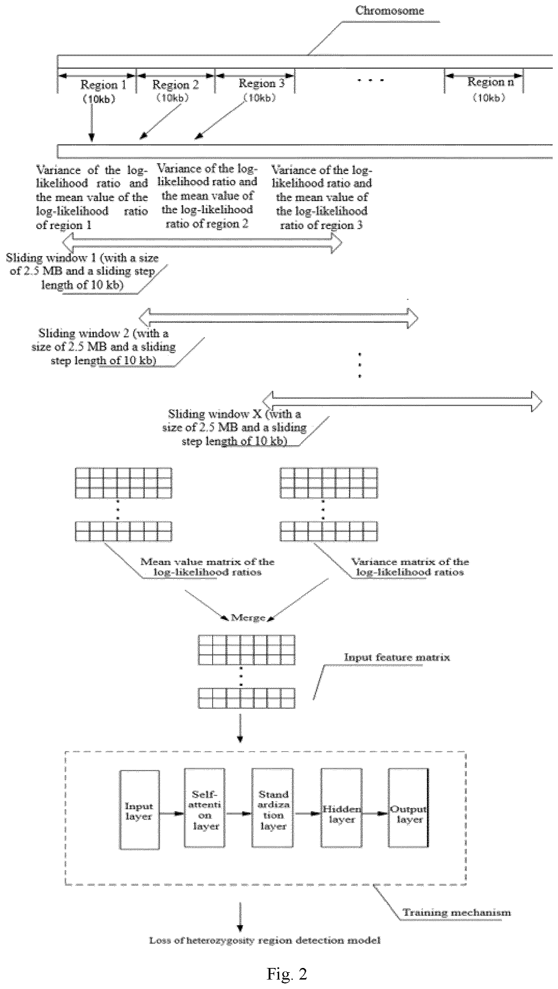

In the process of inputting the input matrix, as shown in Fig. 2, the input matrix is inputted one line at a time; a single input of one line is a training, which is repeated for iterative training until all training is completed.

Further, in the above iterative training process, the activation function selected is Relu function, as shown in the following formula 1:

$$f(x) = max(0, x) \qquad \text{formula 1.}$$

Exemplarily, the above LOH region model is a Recurrent Neural Network (RNN) model, and specifically, can be a BGRU neural network model.

Example 1: The above steps are specifically explained by a specific example below:

Assuming that the first data includes 3X, 1X, 0.5X and 0.1X genome sequencing data, and these genome sequencing data are in BAM format, the region size of each region of the N regions is 10 kb, the sliding window for extracting the feature information is 2.5 MB and the sliding step length is 10 kb:

the 3X, 1X, 0.5X and 0.1X genome sequencing data is firstly obtained; then, the mean value information and the variance information of the N regions including SNP loci in the chromosome corresponding to each set of genome sequencing data are determined for the above 4 sets of genome sequencing data; then, the information of the LOH regions corresponding to the N regions and the association relationship between the mean value information and the variance information of the N regions are determined; next, the input feature matrix for training is determined by using the mean value information and the variance information of the N regions, the LOH region information corresponding to the N regions, and the association relationship between the mean value information and the variance information of the N regions; and finally, the BGRU initial model is iteratively trained by using the input feature matrix to obtain the LOH region detection model.

In the method provided by the embodiment of the present disclosure, one or several sets of different low-depth genome sequencing data obtained based on the high-depth genome sequencing data are obtained at first (the high-depth genome sequencing data includes the data of the LOH regions); then, the information values (the mean value information and the variance information) of each region in the LOH regions in all regions (that is, the N regions) in the chromosome corresponding to the first data are determined; next, the information of the LOH regions corresponding to the N regions and an association relationship between the information values of the N regions are determined, and the input feature matrix for training is determined based on the information of the LOH regions and the association relationship between the information values of each region; and finally, the initial model is iteratively trained based on the input feature matrix to obtain a LOH region detection model. In this way, by acquiring the low-depth genome sequencing data from the high-depth genome sequencing data with known LOH regions, and determining the information value and the feature information of the above low-depth genome sequencing data, then obtaining a training sample, i.e., the input feature matrix, and performing training on the model, the LOH region detection model is finally obtained. Therefore, the LOH region detection model can accurately detect the LOH regions in the chromosomes corresponding to the genome sequencing data by using the low-depth genome sequencing data in the subsequent use process, and can accurately detect the LOH regions without the high-depth genome sequencing data for detection or other complex high-throughput detection modes, thereby greatly improving the detection efficiency.

In another embodiment of the present disclosure, a specific implementation method for determining the information values of each region of the N regions is also provided. Exemplarily, the specific implementation method of "determining the information values of each region of N regions in a chromosome corresponding to the first data" mentioned above includes: calculating likelihood ratio values of each SNP locus in the chromosome corresponding to the first data; calculating log-likelihood information of each SNP locus based on the likelihood ratio values of each SNP locus; dividing, according to the preset range, the chromosome corresponding to the first data above into N regions and determining the information values of each region of the N regions based on the log-likelihood information of each SNP locus above.

Exemplarily, the above likelihood ratio values include a first likelihood ratio value for haplotype and a second likelihood ratio value for diploidtype.

Exemplarily, calculating the likelihood ratio value of each SNP locus in the chromosome corresponding to the first data includes: calculating the first likelihood ratio value for haplotype of each SNP locus in the chromosome corresponding to the first data and the second likelihood ratio value for diploidtype of each SNP locus in the chromosome corresponding to the first data.

Exemplarily, the above SNP loci are known SNP loci. It should be noted that, non-SNP loci do not participate in the above calculation process.

In one example, the above method for calculating the first likelihood ratio value for haplotype is shown by the following formula 2:

$$Pmonosomy(A \wedge B) = f(AB)$$

formula 2.

The above method for calculating the second likelihood ratio value for diploidtype is shown by the following formula 3:

$$Pdisomy(A \wedge B) = 1/2 \, f(AB) + 1/2 f(A) f(B)$$

formula 3.

The A and B mentioned above refer to alleles in the population, and $f(A)$ and $f(B)$ refer to the frequencies of allele A and allele B in the population. A and B are the SNP loci.

Exemplarily, the above log-likelihood information can be a log-likelihood ratio.

Exemplarily, since the above likelihood ratio values are divided into the first likelihood ratio value for haplotype and the second likelihood ratio value for diploidtype, the log-likelihood ratio of each SNP locus is calculated based on the first likelihood ratio value for haplotype and the second likelihood ratio value for diploidtype.

In one example, the formula 4 for calculating the log-likelihood ratio for each SNP locus above is as follows:

$$log - likelihood \; ratio = log \frac{Pmonosomy(A \wedge B)}{Pdisomy(A \wedge B)}$$

formula 4.

Exemplarily, as mentioned above, the information values include the variance information and the mean value information.

Exemplarily, the preset range can be: the preset range of data of the information value for a single calculation divided based on the size of the number that can be observed or calculated at a single time. For example, the above preset range can be 10 kb.

It can be understood that, after the likelihood ratio values and the log-likelihood ratio are obtained, the chromosome is divided into N regions based on the preset range, and the mean value of the log-likelihood ratios and the variance of the log-likelihood ratio of all SNP loci in each region of the N regions are calculated based thereon.

Further, the calculation method above is mainly based on a known Linkage Disequilibrium (LD) pattern in the alleles observed on reads to quantify the probability that two sequencing reads originate from the same chromosome. The allele frequencies and the LD mode from a large population (thousand genome sequencing data) reference panel are used to explain the potential relationship between sparse reads.

In another embodiment of the present disclosure, a specific implementation method of how to encode the LOH regions to facilitate subsequent determination of the input feature matrix is also provided. Exemplarily, prior to "determining the feature information of the N regions, and determining an input feature matrix based on the feature information of the N regions and the information values of the N regions", the specific implementation also includes: dividing the above N regions into X unit regions; detecting the central locus of the above X unit regions; and adding coding information to each unit region of the above X unit regions based on the detection result.

Exemplarily, the X unit regions include at least one region of the N regions, and the X unit regions have overlapping regions therebetween.

Exemplarily, the coding information is used to indicate that each unit region above includes a LOH region.

Exemplarily, as can be seen from the foregoing, the method of determining the feature information above can be obtained through the sliding window. Therefore, the detection of the central locus of the N regions can also be completed by the sliding window, such as a sliding window with a size of 2.5 MB and a sliding step length of 10 kb.

Further, the N regions are divided into X unit regions, the size of each unit region can be consistent with the size of the sliding window. That is, the above process of dividing the N regions can be completed by the sliding window.

Example 2: As shown in Fig. 2, the size of the sliding window is 2.5 MB and the sliding step length is 10 kb, one unit region is generated every time the sliding window slides, and X unit regions are generated when it slides for X times. The N unit regions overlap with each other, and are evenly staggered by the size of 10 kb.

Exemplarily, the codes above may be numbers or letters. In the case that the codes are the numbers, 1 can be used to indicate that the unit region includes the LOH regions, and 0 can be used to indicate that the unit region does not include the LOH region.

In this way, the conditions of the LOH regions in each unit region can be confirmed by dividing the unit region with overlapping parts and detecting the central locus of the unit regions, and the coding information can be added to facilitate subsequent generation of training samples to obtain a model that can confirm the LOH region based on the low-depth data, so as to improve the efficiency of subsequent LOH detection.

In another embodiment of the present disclosure, the details of how to generate the input feature matrix are further informed. Exemplarily, "determining the feature information of the N regions, and determining an input feature matrix based on the feature information of the N regions and the information values of the N regions" mentioned above includes: constructing a first input feature matrix by extracting the first feature information and the first information value corresponding to the above N regions, and constructing a second input feature matrix by extracting the second feature information and the second information value corresponding to the above N regions; and merging the first input feature matrix and the second input feature matrix to generate the input feature matrix.

Exemplarily, the first feature information includes the association relationship between the information of the LOH regions corresponding to N regions and the mean value of the above log-likelihood ratios in the N regions, and the second feature information includes the association relationship between the information of the LOH regions corresponding to N regions and the variance of the above log-likelihood ratios in the N regions.

Exemplarily, the first information value includes the mean value of the above log-likelihood ratios in the N regions, and the second information value includes the variance of the above log-likelihood ratios in the N regions.

In one example, as mentioned above, the method of obtaining the first feature information of the N regions and the second feature information of the N regions above can be obtained by sliding the sliding window on the chromosome.

Example 3: when each region of the N regions is 10 kb, the size of the above sliding window is 2.5 M, and the sliding step length is 10 KB, the sliding window slides on the chromosome, as shown in Fig. 2. Since the size of each window is 2.5 MB, each time the sliding window slides, the mean value of the log-likelihood ratios of 250 regions, the association relationship between the mean values of the log-likelihood ratios of 250 regions and whether the sliding center locus includes the coding information of the LOH regions (that is, the first information value and the first feature information above) are obtained. At the same time, the variances of the log-likelihood ratios of 250 regions, the association relationship between the variances of the log-likelihood ratios of 250 regions, and whether the sliding center locus includes the coding information of the LOH regions (that is, the second information value and the second feature information mentioned above) are also obtained.

After final sliding for X times, an X×1×250 first input feature matrix (the mean value matrix of the log-likelihood ratios) and an X×1×250 second input feature matrix (the variance matrix of the log-likelihood ratios) are obtained.

Then, the above two matrices are combined into a three-dimensional matrix, that is, an X×2×250 input feature matrix.

In this way, by synthesizing the feature values, the association relationship between the feature values and the information of the LOH regions corresponding to the N regions, the training sample, i.e., the input feature matrix is finally obtained. The model obtained by the subsequent training of the input feature matrix can be used to receive the low-depth genome sequencing data to complete the accurate detection of the LOH regions, which improves the detection efficiency of the LOH regions.

In another embodiment of the present disclosure, the specific method of obtaining M sets of first data is also informed. Exemplarily, the specific implementation method of "obtaining the first data" mentioned above includes: extracting, in the case that second data is obtained, M sets of first data in the first format from the second data.

Exemplarily, the above second data is the high-depth genome sequencing data, and the above first data is the low-depth genome sequencing data.

Exemplarily, the data amount of the first data is less than the data amount of the second data.

Exemplarily, the second data may be the second data in the second format.

Further, the first format and the second format may be the same or different, which is not limited by the embodiments of the present application. For example, the above second format can be fastq format, cram format, Bam format and sam format.

In one example, the above extraction of the M sets of first data in the first format from the second data can be completed with a Picard tool.

It should be noted that, in general, the Picard tool can be used to extract the first data in the second data. Further, in the case that the first format and the second format are different, when the Picard tool is used to extract the first data in the second data, the second format of the data can be converted to the first format while extracting the data.

For example, the current second data is 30X genome sequencing data. At this time, the Picard tool is used to extract genome sequencing data with different depths from 30X genome sequencing data successively, extracting 4 sets of 3X, 1X, 0.5X and 0.1X data, respectively, and synchronously converting the data from the CRAM format to the BAM format.

In another embodiment of the present disclosure, the specific detection process after obtaining the LOH region detection model is also informed. The method includes: receiving third data to be detected; detecting the third data by using the LOH region detection model, and determining the detection information of the LOH regions in chromosomal loci corresponding to the third data; and confirming the results of the LOH regions corresponding to the third data based on the detection information.

Exemplarily, the above third data is the low-depth genome sequencing data.

Exemplarily, the above detection information is used to indicate the possibility of the LOH regions in chromosomal locus data corresponding to the third data.

It can be understood that in the above training process, the initial model is trained by using the data information of the LOH regions and the data information of the non-LOH regions to generate the LOH region detection model. Therefore, when the LOH region detection model detects the third data, the model can detect the LOH region in the third data. Specifically, each locus corresponding to the third data can be detected, and then the possibility value that each locus in the third data is located in the LOH region is used to indicate whether the locus belongs to the LOH region.

Example 4: In the case that the information of the LOH regions and the information of the non-LOH regions is represented by 0 or 1, after the third data is input into the LOH region detection model, any decimal number in [0,1] will be outputted correspondingly for each locus. The closer the value is to 1, the more likely it is to be determined that the locus exists in the LOH region. When the output values of the continuous loci in the chromosome corresponding to the third data are all greater than 0.6, the region composed of these continuous loci is the LOH region.

In another embodiment of the present disclosure, the specific process of confirming the results of the LOH region corresponding to the third data is also informed. The specific implementation of the "confirming the results of the LOH regions corresponding to the third data based on the detection information" includes: confirming suspected LOH regions in the chromosome corresponding to the third data based on the above detection information; screening out exposed Unmask regions in the suspected LOH regions to obtain confirmed LOH regions; and merging the confirmed LOH regions and confirming the results of the LOH regions corresponding to the third data above.

It can be understood that in the above example 4, the region composed of continuous loci is essentially the suspected LOH region, and after obtaining the suspected LOH region, the location of all LOH regions can be located. If the location distance of the suspected LOH regions on the same chromosome is less than 1 MB, these regions are merged. The unmask region of the chromosome region is removed in the merged region.

It should be noted that if the unmask region is located inside the LOH regions, the LOH regions are regarded as two LOH regions. Finally, if the region is larger than 3 MB, it is determined as a LOH region and the result is outputted.

The above solution is further described below with a practical example in the embodiment of the present disclosure. The model generation process of the practical example can be corresponding to Fig. 2:

As shown in Table 1, 10 cases of high-depth genome sequencing data (i.e., second data) are used; the 30X depth data has a total of 10 LOH regions, totaling about 122.73 MB, in CRAM format (i.e., second format). The 30X genome CRAM data are grouped and extracted into 4 sets of 3X, 1X, 0.5X and 0.1X data by using the Picard tool and converted into BAM files.

The mean value and the variance of the log-likelihood ratios of N regions per 10 KB range on each chromosome corresponding to the BAM files of each set of data are obtained by the above formulas 2, 3, and 4. Then, a sliding window with a size of 2.5 MB and a step length of 10 KB is used to slide on the chromosome to obtain the mean value and the variance of the log-likelihood ratios of 250 regions in each window to construct a $2\times250$ feature matrix, and the window slides for Z times. At the same time, it is determined whether the central locus of the sliding window is included in the identified LOH region, and the information of the LOH region is recorded together. Based on this information, all the loci on the chromosome are divided into two categories: those in the LOH region and those not in the LOH region. It is also necessary to record the association relationship between different variances or different mean values in each window.

Based on the above information, a $Z\times1\times250$ matrix is constructed by using the information of the LOH regions corresponding to the N regions, the association relationship between the mean values of the log-likelihood ratios in the N regions and the mean values of the log-likelihood ratios in the N regions. A $Z\times1\times250$ matrix is constructed by using the information of the LOH regions corresponding to the N regions, the association relationship between the variances of the log-likelihood ratios in the N regions and the variances of the log-likelihood ratios in the N regions. The above two matrices are merged into a $Z\times2\times250$ three-dimensional input feature matrix.

The above three-dimensional feature matrix is taken as a model input matrix. The LOH region detection model is constructed by a customized neural network.

Table 1

| Sample | LOH region | | | |
|---|---|---|---|---|
| | Chr | Start | End | Size (MB) |
| Sample 1 | 10 | 83689905 | 88832673 | 5.14 |
| Sample 2 | 12 | 102574695 | 108343641 | 5.77 |
| Sample 3 | 7 | 122381633 | 130929938 | 8.55 |
| Sample 4 | 1 | 81001158 | 87342491 | 6.34 |
| Sample 5 | 7 | 76139731 | 98494568 | 22.35 |
| Sample 6 | 5 | 98454988 | 107968822 | 9.51 |
| Sample 7 | 7 | 89568718 | 98528006 | 8.96 |
| Sample 8 | 17 | 38317185 | 58003268 | 19.69 |
| Sample 9 | 1 | 211199823 | 234941343 | 23.74 |
| Sample 10 | 2 | 71476234 | 84154322 | 12.68 |
| | Total | | | 122.73 |

After the LOH region detection model is obtained, 306 samples (including 781 known LOH regions) are used in the model to verify the accuracy of the model.

After the suspected LOH regions are obtained in the input model, the locations of all the LOH regions are located, and the regions whose locations are less than 1 MB apart on the same chromosome are merged. The unmask region of the chromosome region is removed from the merged region. If the unmask region is located inside the LOH regions, the LOH regions are regarded as two LOH regions. If the region is larger than 3 MB, it is judged as a LOH region and the result is outputted.

Finally, the region where the identified LOH region has 50% intersection with the known LOH region is defined as the accurately identified region (as shown by the following formula 4). 781,781,780 and 750 LOH regions are identified at different depths of 3X, 1X, 0.5X and 0.1X, respectively, and the identification accuracy remains above 50%, as shown in Table 2.

| | LOH | | |
|---|---|---|---|
| Depth | Known region | Identified region | Identification accuracy |
| 3X | 781 | 781 | 100% |
| 1X | 781 | 781 | 100% |
| 0.5X | 781 | 780 | 99.9% |
| 0.1X | 781 | 750 | 96.0% |

$$overlap\% = \frac{LOH\_length_{CMA} \ \cap \ LOH\_length_{predict}}{LOHl\_length_{CMA}}\% \qquad \text{Formula 4}$$

Wherein LOH_lengthCMA represents the length of the LOH region result obtained through CMA experiments provided by the thousand genomes sequencing data, and LOH_lengthpredict represents the length of the LOH region result predicted by the model.

The embodiment of the present disclosure also provides a computer-readable storage medium having a computer program stored thereon, the program, when executed by a processor, implements the method for determining training rules described in the embodiment of the present disclosure. For example, the steps of the method shown in Fig. 1 can be performed.

The embodiment of the present disclosure provides a computer program product which includes instructions. The instructions, when executed by a processor, implement the steps of the method shown in Fig. 1.

It should be noted that although the operations of the method of the present disclosure are described in a particular order in the drawings, it is not required or implied that the operations must be performed in the particular order, or that all of the operations shown must be performed to achieve the desired results.

Fig. 3 is a schematic block diagram of a training apparatus for a loss of heterozygosity region detection model provided by an embodiment of the present disclosure. Referring to Fig. 3, the apparatus includes an acquisition module 601, a determination module 602, and a training module 603.

The acquisition module 601 is configured to acquire first data, wherein the first data is low-depth genome sequencing data obtained based on high-depth genome sequencing data; the first data including M types of low-depth genome sequencing data, where M is a positive integer.; and the high-depth genome sequencing data including the data of LOH regions.

The determination module 602 is configured to determine information values of each region of N regions in a chromosome corresponding to the first data obtained by the acquisition module, wherein the information values include mean value information and variance information of the above regions; the N regions including heterozygous regions and the LOH regions, where N is a positive integer.

The determination module 602 is configured to determine feature information of the N regions, and determine an input feature matrix based on the feature information of the N regions and the information values of the N regions, wherein the feature information includes the information of the LOH regions corresponding to the N regions and an association relationship between the information values of each region of the N regions.

The training module 603 is configured to perform iterative training on an initial model based on the input feature matrix determined by the determination module to obtain a LOH region detection model, wherein the initial model is determined based on a self-attention allocation algorithm.

In one embodiment, the above apparatus includes a calculation module, the calculation module is configured to:

    calculate the likelihood ratio value of each SNP locus in the chromosome corresponding to the first data, wherein the above likelihood ratio values include a first likelihood ratio value for haplotype and a second likelihood ratio value for diploidtype; and
    calculate the log-likelihood information of each SNP locus based on the likelihood ratio value of each SNP locus.

The determination module 602 is configured to divide the chromosome corresponding to the first data above into N regions based on the preset range, and determine the information values of each region of the N regions based on the log-likelihood information of each SNP locus above.

In one embodiment, the calculation module is also configured to calculate the first likelihood ratio value for haplotype of each SNP locus in the chromosome corresponding to the first data and the second likelihood ratio value for diploidtype of each SNP locus in the chromosome corresponding to the first data.

In one embodiment, the determination module 602 is specifically configured to:

divide the above N regions into X unit regions, wherein the X unit regions include at least one region of the N regions, and wherein the X unit regions have overlapping regions therebetween;
detect central locus of the above X unit regions; and
add coding information to each unit region of the above X unit regions based on the detection result, the coding information being used to indicate that each unit region above includes a LOH region.

In one embodiment, the determination module 602 is specifically configured to:

construct a first input feature matrix by extracting first feature information and first information value corresponding to the above N regions, and construct a second input feature matrix by extracting second feature information and second information value corresponding to the above N regions, wherein the first feature information includes an association relationship between the information of the LOH regions corresponding to N regions and a mean value of the above log-likelihood ratios in the N regions, and wherein the second feature information includes an association relationship between the information of the LOH regions corresponding to N regions and a variance of the above log-likelihood ratios in the N regions; wherein the first information value includes the mean value of the above log-likelihood ratios in the N regions, and wherein the second information value includes the variance of the above log-likelihood ratios in the N regions; and
merge the first input feature matrix and the second input feature matrix to generate the input feature matrix.

In one embodiment, the acquisition module 601 is specifically configured to extract, in the case that the second data is obtained, M sets of the first data in the first format from the second data, wherein: the above second data is the high-depth genome sequencing data, and the above first data is the low-depth genome sequencing data.

In one embodiment, the apparatus also includes:

a receiving module configured to receive third data to be detected, wherein the third data is the low-depth genome sequencing data; and
a determination module 602 configured to detect the third data by using the LOH region detection model, determine the detection information of the LOH regions in chromosomal loci corresponding to the third data, wherein the above detection information is used to indicate a possibility of the LOH regions in chromosomal locus data corresponding to the third data, and confirm the results of the LOH regions corresponding to the third data based on the detection information.

In one embodiment, the determination module 602 is specifically configured to:

confirm suspected LOH regions in a chromosome corresponding to the third data based on the above detection information;
screen out Unmask regions in the suspected LOH regions to obtain confirmed LOH regions; and
merge the confirmed LOH regions according to a preset distance range and confirm the results of the LOH regions corresponding to the above third data.

In the embodiment of the present disclosure, the apparatus for training the loss of heterozygosity region detection model firstly obtains one or several sets of different low-depth genome sequencing data obtained based on the high-depth genome sequencing data (the high-depth genome sequencing data includes the data of the LOH regions); then, determines the information values (mean value information and variance information) of the LOH regions in all regions (that is, N regions) in the chromosomes corresponding to the first data; next, determines the information of the LOH regions corresponding to the N regions and the association relationship between the information values of the N regions, and determines the input feature matrix for training based on the information of the LOH regions and the association relationship between the information values of each region; and finally, obtains the LOH region detection model by performing iterative training on an initial model based on the input feature matrix. In this way, by acquiring the low-depth genome sequencing data from the high-depth genome sequencing data with known LOH regions, and determining the information value and the feature information of the above low-depth genome sequencing data, then obtaining a training sample, i.e., the input feature matrix, and performing training on the model, the LOH region detection model is finally obtained. Therefore, the LOH region detection model can accurately detect the LOH regions in the chromosomes corresponding to the genome sequencing data by using the low-depth genome sequencing data in the subsequent use process, and can accurately detect the LOH regions without the high-depth genome sequencing data for detection or other complex high-throughput detection modes, thereby greatly improving the detection efficiency.

It should be understood that the units recorded in the training apparatus for the loss of heterozygosity region detection model correspond to the steps in the method described in the drawings. Therefore, the operations and features described above for the method are also applicable to the training apparatus for the loss of heterozygosity region detection model, a resource access apparatus and the units included therein, and will not be described here. The training apparatus for the loss of heterozygosity region detection model and the resource access apparatus can be pre-implemented in a browser or other security applications of the computer device, or can be loaded into the browser or the security applications of the computer device by downloading and other modes. The corresponding units in the training apparatus for the loss of heterozygosity region detection model and the resource access apparatus can be cooperated with the units in the computer device to realize the solution of the embodiments of the present disclosure.

For several modules or units mentioned in the detailed description above, this division is not mandatory. In fact, according to the embodiments of the present disclosure, the features and functions of two or more modules or units described above may be embodied in one module or unit. Conversely, the features and functions of one module or unit described above can be further divided into multiple modules or units and embodied.

It should be noted that, for the details not disclosed in the training apparatus for the loss of heterozygosity region detection model and the resource access apparatus in the embodiments of the present disclosure, please refer to the details disclosed in the above embodiments of the present disclosure and will not be repeated here.

Referring to Fig. 4 below, Fig. 4 shows a schematic diagram of a structure of a computer device suitable for implementing the embodiments of the present disclosure. As shown in Fig. 4, the computer system 1700 includes a Central Processing Unit (CPU) 1701 which can execute various appropriate actions and processing based on programs stored in a Read-Only Memory (ROM) 1702 or programs loaded into a Random Access Memory (RAM) 1703 from a storage part 1708. Various programs and data required for the operation instructions of the system are also stored in the RAM 1703. The CPU 1701, the ROM 1702 and the RAM 1703 are mutually connected through a bus 1704. An Input/Output (I/O) interface 1705 is also connected to the bus 1704.

The following components are connected to the I/O interface 1705: an input part 1706 including a keyboard, a mouse and the like; an output part 1707 including a Cathode Ray Tube (CRT), a Liquid Crystal Display (LCD), a loudspeaker and the like; a storage part 1708 including a hard disk and the like; and a communication part 1709 including network interface cards such as a LAN card, a modem and the like. The communication part 1709 executes communication processing through a network such as Internet. A driver 1710 is also connected to the I/O interface 1705 as required. A removable medium 1711, such as a disk, a CD, a magneto-optical disc and a semiconductor memory, is installed on the driver 1710 as required, so that the computer programs read therefrom to be installed into the storage part 1708 as required.

Particularly, based on the embodiments of the present disclosure, the process described with reference to the flow chart in Fig. 1 can be realized as a computer software program. For example, an embodiment of the present disclosure includes a computer program product, which includes a computer program carried on a computer-readable medium. The computer programs include program codes used for executing the method shown in the flow chart. In such an embodiment, the computer programs include program codes for executing the method shown in the flow chart. In such an embodiment, the computer programs can be downloaded and installed from the network through the communication part 1709, and/or installed from the removable medium 1711. When the computer programs are executed by the Central Processing Unit (CPU) 1701, the functions defined in the system of the present disclosure are performed.

It should be noted that the computer-readable medium shown in the present disclosure can be a computer-readable signal medium or a computer-readable storage medium or any combination of the above. The computer-readable storage medium, for example, can be, but not limited to, electrical, magnetic, optical, electromagnetic, infrared, or semiconductor systems, apparatuses or devices, or any combination of the above. More specific examples of the computer-readable storage medium may include, but not limited to: electrical connection with one or more wires, a portable computer disk, a hard disk, a Random Access Memory (RAM), a Read-Only Memory (ROM), an Erasable Programmable Read-Only Memory (EPROM or flash memory), an optical fiber, a portable Compact Disk Read-Only Memory (CD-ROM), an optical storage device, a magnetic storage device, or any suitable combination of the above. In the present disclosure, the computer-readable storage medium may be any tangible medium containing or storing a program, which can be used by or in combination with an instruction execution system, apparatus or device. In the present disclosure, the computer-readable signal medium may include a data signal propagated in a baseband or as part of a carrier, in which the computer-readable program codes are carried. This propagated data signal may take many forms, including but not limited to electromagnetic signals, optical signals, or any suitable combination of the above. The computer-readable signal medium may also be any computer-readable medium, such as a computer-readable storage medium. The computer-readable medium may send, propagate or transmit a program used by or used in combination with the instruction execution system, apparatus or device. The program codes contained on the computer-readable medium can be transmitted in any appropriate medium, including, but not limited to: wireless, wire, optical cable, RF, etc., or any suitable combination of the above.

The flow charts and block diagrams in the drawings show the realizable architectures, functions and operation instructions of the system, the method and the computer program product according to various embodiments of the present disclosure. In this regard, each block in the flow charts or block diagrams can represent a module, a program segment or a part of codes; which includes one or more executable instructions for realizing specified logical functions. It should also be noted that in some alternative implementations, the functions indicated in the blocks may also occur in a sequence different from that indicated in the drawings. For example, two blocks that are indicated by connection can actually be executed essentially concurrently, and sometimes can also be executed in reverse order, depending on the involved functions. It should also be noted that each block in the block diagrams and/or flow charts and combinations of the blocks in the block diagrams and/or flow charts can be implemented by a dedicated hardware-based system that executes specific functions or operation or by combinations of dedicated special hardware and computer instructions.

The units or modules described in the embodiments of the present disclosure can be realized by software or hardware. The described units or modules may also be set in a processor, for example, it may be described that a processor includes a first receiving module, a second receiving module, and a sending module; wherein, the names of these units or modules do not, in some cases, constitute a limitation of the units or modules.

On the other hand, the present disclosure also provides a computer-readable storage medium, which may be included in the electronic device described in the above embodiment or may exist separately and not be assembled into the electronic device. The computer-readable storage medium above stores one or more programs used by one or more processors to execute the training rule determination method and an unethical business identification method described in the present disclosure.

The above description is only the explanation of the preferred embodiments of the present disclosure and the used technical principles. Those skilled in the art shall understand that the disclosure scope involved in the present disclosure is not limited to the technical solutions formed by the specific combinations of the above technical features, and also covers other technical solutions formed by any combination of the above technical features or equivalent features without departing from the concept of the present disclosure, e.g., the technical solutions formed by interchanging the above features and (but not limited to) technical features with similar functions disclosed the present disclosure.

**Claims**

1. A method for training a Loss of Heterozygosity (LOH) region detection model, comprising:

   acquiring first data, wherein the first data is low-depth genome sequencing data acquired based on high-depth genome sequencing data, the first data comprising M types of low-depth genome sequencing data, where M is a positive integer; and the high-depth genome sequencing data comprising data of LOH regions;
   determining information values of each region of N regions in a chromosome corresponding to the first data, wherein the information values comprise mean value information and variance information of the region; the N regions comprising heterozygous regions and the LOH regions, where N is a positive integer;
   determining feature information of the N regions, and determining an input feature matrix based on the feature information of the N regions and the information values of the N regions, wherein the feature information comprises information of the LOH regions corresponding to the N regions and an association relationship between the information values of each region of the N regions; and
   performing iterative training on an initial model based on the input feature matrix to obtain a LOH region detection model, wherein the initial model is determined based on a self-attention allocation algorithm.

2. The method according to claim 1, wherein said determining the information values of each region of the N regions in the chromosome corresponding to the first data comprises:

   calculating likelihood ratio values of each Single Nucleotide Polymorphism (SNP) locus in the chromosome corresponding to the first data, wherein the likelihood ratio values comprise a first likelihood ratio value for haplotype and a second likelihood ratio value for diploidtype;
   calculating log-likelihood information of each SNP locus based on the likelihood ratio values of each SNP locus; and
   dividing, according to a preset range, the chromosome corresponding to the first data into N regions and determining the information values of each region of the N regions based on the log-likelihood information of each SNP locus.

3. The method according to claim 2, wherein said calculating the likelihood ratio values of each SNP locus in the chromosome corresponding to the first data comprises:
   calculating the first likelihood ratio value for haplotype of each SNP locus in the chromosome corresponding to the first data and the second likelihood ratio value for diploidtype of each SNP locus in the chromosome corresponding to the first data.

4. The method according to claim 1, further comprising, prior to said determining the feature information of the N regions, and determining the input feature matrix based on the feature information of the N regions and the information values of the N regions:

dividing the N regions into X unit regions, wherein the X unit regions comprise at least one region of the N regions, and wherein the X unit regions have overlapping regions therebetween;
detecting central locus of the X unit regions; and
adding coding information to each unit region of the X unit regions based on detection result, the coding information being used to indicate that each unit region comprises a LOH region.

5. The method according to claim 1, wherein said determining the feature information of the N regions, and determining the input feature matrix based on the feature information of the N regions and the information values of the N regions comprises:

constructing a first input feature matrix by extracting first feature information and first information value corresponding to the N regions, and constructing a second input feature matrix by extracting second feature information and second information value corresponding to the N regions, wherein the first feature information comprises an association relationship between the information of the LOH regions corresponding to N regions and a mean value of the log-likelihood ratios of the N regions, wherein the second feature information comprises an association relationship between the information of the LOH regions corresponding to N regions and a variance of the log-likelihood ratios of the N regions, wherein the first information value comprises a mean value of the log-likelihood ratios of the N regions, and wherein the second information value comprises a variance of the log-likelihood ratios of the N regions; and
merging the first input feature matrix and the second input feature matrix to generate the input feature matrix.

6. The method according to claim 1, wherein said obtaining the first data comprises:
extracting, in the case that second data is obtained, M sets of the first data from the second data, wherein:

the second data is the high-depth genome sequencing data; and
the first data is the low-depth genome sequencing data.

7. The method according to claim 1, further comprising:

receiving third data to be detected, wherein the third data is the low-depth genome sequencing data;
detecting the third data by using the LOH region detection model, and determining detection information of the LOH regions in chromosomal loci corresponding to the third data, wherein the detection information is used to indicate a possibility of the LOH regions in chromosomal locus data corresponding to the third data; and
confirming results of the LOH regions corresponding to the third data based on the detection information.

8. The method according to claim 7, wherein said confirming the results of the LOH regions corresponding to the third data based on the detection information comprises:

confirming suspected LOH regions in a chromosome corresponding to the third data based on the detection information;
screening out unmask regions in the suspected LOH regions to obtain confirmed LOH regions; and
merging the confirmed LOH regions according to a preset distance range and confirming the results of the LOH regions corresponding to the third data.

9. An apparatus for training a loss of heterozygosity region detection model, comprising:

an acquisition module configured to acquire first data, wherein the first data is low-depth genome sequencing data acquired based on high-depth genome sequencing data, the first data comprising M types of low-depth genome sequencing data, where M is a positive integer; and the high-depth genome sequencing data comprising the data of LOH regions;
a determination module configured to determine information values of each region of N regions in a chromosome corresponding to the first data acquired by the acquisition module, wherein the information values comprise mean value information and variance information of the region, the N regions comprising heterozygous regions and the LOH regions, where N is a positive integer;

a determination module configured to: determine feature information of the N regions, and determine an input feature matrix based on the feature information of the N regions and the information values of the N regions, wherein the feature information comprises information of the LOH regions corresponding to the N regions and an association relationship between the information values of each region of the N regions; and

a training module configured to perform iterative training on an initial model based on the input feature matrix determined by the determination module to obtain a LOH region detection model, wherein the initial model is determined based on a self-attention allocation algorithm.

10. A computer device, comprising:

a memory,
a processor; and
a computer program stored on the memory and executable on the processor, wherein the computer program, when executed by a processor, implements the method according to any one of claims 1 to 8.

11. A computer-readable storage medium, having a computer program stored thereon, wherein the computer program, when executed by a processor, implements the method according to any one of claims 1 to 8.

12. A computer program product, comprising instructions, wherein the instructions, when executed by a processor, implement the method according to any one of claims 1 to 8.

acquiring first data      301

determining information values of each region of N regions
in a chromosome corresponding to the first data      302

determining feature information of the N regions, and determining an input
feature matrix based on the feature information of the N regions and the
information values of the N regions      303

performing iterative training on an initial model based on the input feature
matrix to obtain a LOH region detection model      304

Fig. 1

Fig. 2

Apparatus for training a loss
of heterozygosity region
detection model

Acquisition module ——— 601

Determination
module ——— 602

Training module ——— 603

Fig. 3

1700

CPU 1701    ROM 1702    RAM 1703

1704

I/O interface 1705

Input part 1706    Output part 1707    Storage part 1708    Communication part 1709    Driver 1710

Removable medium 1711

Fig. 4

## INTERNATIONAL SEARCH REPORT

| International application No. |
| --- |
| **PCT/CN2023/101131** |

| A. | CLASSIFICATION OF SUBJECT MATTER |
| --- | --- |

G16B20/30(2019.01)i

According to International Patent Classification (IPC) or to both national classification and IPC

| B. | FIELDS SEARCHED |
| --- | --- |

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CNABS: CNTXT; VEN; USTXT; EPTXT; WOTXT; CNKI; IEEE: 杂合性缺失, 检测, 识别, 模型, 神经网络, 训练, 低深度, 基因, 测序, 均值, 方差, 关系, loss of heterozygosity, LOH, detect, recognise, model, neural network, train, low depth, gene, sequence, mean value, variance, relationship

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| A | WO 2022226186 A1 (PERSONALIS INC.) 27 October 2022 (2022-10-27) description, paragraphs 9-61 | 1-12 |
| A | CN 112885406 A (SHENZHEN YUCE BIO TECHNOLOGY CO., LTD.) 01 June 2021 (2021-06-01) description, paragraphs 6-46 | 1-12 |
| A | US 2021366570 A1 (SOPHIA GENETICS S/A) 25 November 2021 (2021-11-25) entire document | 1-12 |
| A | CN 115747334 A (BEIJING YOUXUN MEDICAL DEVICES CO., LTD.) 07 March 2023 (2023-03-07) entire document | 1-12 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 February 2024** | **02 March 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/101131**

| Patent document cited in search report | | | Publication date (day/month/year) | Patent family member(s) | | | Publication date (day/month/year) |
|---|---|---|---|---|---|---|---|
| WO | 2022226186 | A1 | 27 October 2022 | None | | | |
| CN | 112885406 | A | 01 June 2021 | CN | 112885406 | B | 31 January 2023 |
| US | 2021366570 | A1 | 25 November 2021 | EP | 3658687 | A1 | 03 June 2020 |
| | | | | WO | 2019020652 | A1 | 31 January 2019 |
| | | | | US | 11830579 | B2 | 28 November 2023 |
| CN | 115747334 | A | 07 March 2023 | None | | | |

Form PCT/ISA/210 (patent family annex) (July 2022)